# EUROPEAN PATENT SPECIFICATION

(11) **EP 3 859 332 B1**
(45) Date of publication and mention of the grant of the patent: **23.08.2023**
(21) Application number: 19865242.2
(22) Date of filing: 27.09.2019
(51) Int. Cl.: C07K 16/18, C07K 16/44, G01N 33/72

(54) **GLYCATED HEMOGLOBIN (%) ASSAY METHOD**
TESTVERFAHREN FÜR GLYKIERTES HÄMOGLOBIN (% )
PROCÉDÉ DE DOSAGE D'HÉMOGLOBINE GLYQUÉE (%)

(30) Priority: 28.09.2018 JP 2018184017
(43) Date of publication of application: 04.08.2021
(73) Proprietor: Sekisui Medical Co., Ltd., Tokyo 103-0027 (JP)
(72) Inventor: OTA, Mieko, Tokyo 103-0027 (JP); MIYAZAKI, Osamu, Tokyo 103-0027 (JP); SEO, Satoko, Tokyo 103-0027 (JP); YAMAMOTO, Mitsuaki, Tokyo 103-0027 (JP); NISHIO, Tomohisa, Tokyo 103-0027 (JP)
(74) Representative: Hoffmann Eitle
(86) International application number: PCT/JP2019/038076
(87) International publication number: WO 2020/067396

(56) References cited:
- WO-A1-02/18953
- WO-A1-02/095407
- WO-A1-2014/112318
- JP-A- H0 735 752
- JP-A- H0 954 088
- JP-A- H1 010 127
- JP-A- 2002 365 290
- JP-A- 2013 076 713
- JP-A- 2013 511 717
- JP-A- 2016 031 250
- JP-A- 2016 031 250
- Yelton D E ET AL: "Use of Monoclonal Anti-mouse Immunoglobulin to Detect Mouse Antibodies", Hybridoma, 1 January 1981 (1981-01-01), pages 5-11, XP55918739, United States DOI: 10.1089/hyb.1.1981.1.5 Retrieved from the Internet: URL:https://doi.org/10.1089/hyb.1.1981.1.5 [retrieved on 2022-05-06]
- Anonymous: "Secondary Antibodies", Internet Article, 9 August 2019 (2019-08-09), XP055918657, Retrieved from the Internet: URL:https://www.bio-rad-antibodies.com/sta tic/2019/secondary/secondaries-antibodies- guide.pdf [retrieved on 2022-05-06]

## Description

### TECHNICAL FIELD

The present invention relates to a glycated human hemoglobin (%) assay method and assay reagent using an immune reaction. More specifically, the present invention relates to a glycated human hemoglobin (%) assay method and assay reagent using a plurality of monoclonal antibodies.

### BACKGROUND ART

Hemoglobin A1c (hereinafter also simply referred to as HbA1c) is glycated hemoglobin in which glucose is non-enzymatically bound to an α-amino group of an amino acid residue at a β-chain N-terminal of hemoglobin. Hemoglobin is composed of two α chains and two β chains and has a heterotetramer structure. It is known that a ratio of HbA1c in blood reflects a relatively long-term glycemic control state of diabetes, and measuring HbA1c is clinically highly meaningful for knowing the glycemic control state. The following techniques are known as immunological assay methods for HbA1c.

Patent Document 1 relates to an HbA1c (%) assay method in which unsensitized latex is reacted with a sample containing HbA1c and an anti-HbA1c antibody is then reacted to measure agglutination of the latex adsorbed by HbA1c via the anti-HbA1c antibody. In this assay method, only one type of monoclonal antibody to HbA1c is used.

Patent Document 2 relates to an agglutination immunoassay method in which HbA1c in a sample is adsorbed to insoluble carrier particles such as latex, reacted with an anti-HbA1c monoclonal antibody (first antibody), and then further reacted with a second antibody selectively bound to the monoclonal antibody to selectively agglutinate insoluble carrier particles. Although two types of antibodies are used in this assay, the monoclonal antibody is only the first antibody serving as the anti-HbA1c monoclonal antibody, and the second antibody which binds to the anti-HbA1c monoclonal antibody is a polyclonal antibody, not a monoclonal antibody.

An HbA1c (%) assay reagent including a first reagent containing latex and a second reagent containing a bound substance of an anti-human HbA1c monoclonal antibody and an anti-mouse IgG antibody is known as a conventional product (Non-Patent Documents 1, 2). According to this assay reagent, HbA1c in blood is adsorbed to the latex, the bound substance of the antibodies immunologically reacts with HbA1c adsorbed to the latex, and the agglutination of the latex generated in this case is obtained as turbidity.

While normal hemoglobin is a heterotetramer composed of two α chains and two β chains as described above, it is known that abnormal hemoglobin with a portion of the β chain mutated is present in the blood of some Africans and black Americans. Specific examples include HbS in which the sixth Glu from the β-chain N-terminal of hemoglobin (Hb) is mutated to Val, and HbC in which the same sixth Glu is mutated to Lys. Although the number of Japanese with HbS symptom or HbC symptom carrying abnormal hemoglobin having such a mutated sequence is extremely small, such persons still exist in a certain proportion. When the blood of patients with such abnormal hemoglobin symptoms was measured by a conventional HbA1c assay method, it was found that the measurement value of HbA1c (%) (strictly speaking, the measurement value of glycated hemoglobin (%) such as glycated HbS and glycated HbC) becomes higher than that of an HPLC method and accurate measurement cannot be performed (see Comparative Example 2 in Examples described later).

Patent Document 3 describes a method for detecting glycated hemoglobin present in a sample by digesting an endopeptidase-treated human hemoglobin-containing sample with proline-specific endopeptidase and quantifying a level of obtained glycated pentapeptide for the purpose of detecting all glycated hemoglobin in a sample regardless of a form of glycated hemoglobin, i.e., detecting glycated human hemoglobin A, glycated human hemoglobin S, and glycated human hemoglobin C. This method utilizes the fact that digestion of samples containing hemoglobin A, hemoglobin S, and hemoglobin C by proline-specific endopeptidase generates the same glycated pentapeptide in each case of hemoglobin A, hemoglobin S, and hemoglobin C.

The quantification of the glycated pentapeptide is performed by an agglutination inhibition measurement method using a mouse monoclonal anti-HbA1c antibody. However, this method requires two types of peptidase treatment steps, and the operation is complicated. Yelton et al. relates to use of monoclonal anti-mouse immunoglobulin to detect mouse antibodies. JP 2019 031250 relates to a reagent for assaying target protein and assay method using the reagent, JP H09 54088 relates to measuring HbA1c amount with respect to Hba amount.

### CITATION LIST

### PATENT LITERATURE

Patent Document 1: Japanese Laid-Open Patent Publication No. 6-66795
Patent Document 2: Japanese Laid-Open Patent Publication No. 6-167495
Patent Document 3: Japanese Unexamined Patent Application Publication JP 2019 031250 (Horiba Ttd) JP H09 54088 (Kyoto Daiichi KK) (Translation of PCT Application) No. 2011-503632

### NON PATENT LITERATURE

Non-Patent Document 1: Rapidia (registered trademark) Auto HbA1c Attached Document
Non-Patent Document 2: Detamina (registered trademark) LHbA1c Attached Document Yelton et al., 1981, "Use of monoclonal anti-mouse immunoglobulin to detect mouse antibodies", Hybridoma 1(1): 5-11

### SUMMARY

### TECHNICAL PROBLEM

A problem to be solved by the present invention is to provide a glycated human hemoglobin (%) assay method and assay reagent capable of accurate measurement of glycated human hemoglobin (%) in a human sample without being affected by abnormal hemoglobin even when a specimen containing hemoglobin contains abnormal hemoglobin such as HbS or HbC.

### SOLUTION TO PROBLEM

The present invention relates to a glycated human hemoglobin (%) assay method for measuring glycated human hemoglobin (%) in a human sample, comprising steps of:
adsorbing the glycated human hemoglobin in the human sample to an insoluble carrier by bringing the human sample into contact with the insoluble carrier; wherein the insoluble carrier is latex
forming a complex of an anti-glycated human hemoglobin monoclonal antibody, the glycated human hemoglobin, and the insoluble carrier by bringing the glycated human hemoglobin adsorbed to the insoluble carrier into contact with the anti-glycated human hemoglobin monoclonal antibody;
agglutinating the insoluble carrier by bringing the complex into contact with a monoclonal antibody reacting with the anti-glycated human hemoglobin monoclonal antibody and not reacting with human IgG; and
optically detecting a degree of agglutination.

Further aspects of the invention are defined in the claims.

### ADVANTAGEOUS EFFECTS

According to the present invention, accurate immunoassay of glycated human hemoglobin (%) can be performed even when a specimen contains not only normal hemoglobin but also abnormal hemoglobin such as HbS and HbC. According to the present invention, since all the specimens can be measured in the same manner without particularly selecting the abnormal hemoglobin specimens, a reagent suitable for automated continuous measurement can be provided.

### DESCRIPTION

### (Anti-Glycated human Hemoglobin Monoclonal Antibody)

Monoclonal antibodies of the present invention include at least a first monoclonal antibody and a second monoclonal antibody, and these antibodies are used in combination when glycated human hemoglobin (%) is measured. Any monoclonal antibody can be used as the first monoclonal antibody as long as the monoclonal antibody specifically reacts with human glycated hemoglobin. Specifically reacting with human glycated hemoglobin means that the antibody reacts with human glycated hemoglobin and does not substantially react with non-glycated human hemoglobin.

The first monoclonal antibody of the present invention is a monoclonal antibody recognizing a β-chain N-terminal region specific for glycated human hemoglobin such as HbA1c, and since this β-chain N-terminal region is embedded and present inside of a higher-order structure of human hemoglobin, a denaturation treatment of a specimen is usually required for immunoassay of glycated human hemoglobin with an anti-glycated human hemoglobin monoclonal antibody. However, it is considered that since the structure of glycated human hemoglobin is changed by adsorbing glycated human hemoglobin to an insoluble carrier such as latex, the β-chain N-terminal region of glycated human hemoglobin is exposed, which enables the immunoassay with the anti-glycated human hemoglobin antibody.

### (Monoclonal Antibody to Anti-Glycated human Hemoglobin Monoclonal Antibody)

Any monoclonal antibody can be used as the second monoclonal antibody of the present invention as long as the monoclonal antibody has the following properties:
1) Reactive with anti-glycated human hemoglobin monoclonal antibody
2) Not reactive with human IgG

A glycated human hemoglobin (%) assay method using a combination of the first monoclonal antibody and the second monoclonal antibody of the present invention is a glycated human hemoglobin (%) assay method hardly affected by mutation even in the case of a specimen of abnormal human hemoglobin such as HbS and HbC, which are mutants of normal human hemoglobin, contained in a sample. Measurement objects of glycated human hemoglobin of the present invention include glycated HbS and glycated HbC generated by glycation of such abnormal human hemoglobin, as well as normal glycated human hemoglobin (HbA1c). Therefore, an accurate measurement value of glycated human hemoglobin (%) can be obtained even when the specimen contains any glycated human hemoglobin.

A conventional immunoagglutination assay using a polyclonal antibody as a second antibody has a problem that a measurement value of glycated human hemoglobin (%) becomes high and deviates from a true value when abnormal human hemoglobin is present. However, by using a monoclonal antibody to the anti-glycated human hemoglobin monoclonal antibody as the second antibody as in the assay method of the present invention, glycated human hemoglobin (%) can be measured accurately even if abnormal hemoglobin is present in a sample although the reason is not clear.

In this description, when steps of reacting the first monoclonal antibody and the second monoclonal antibody with respective antigens are sequentially performed, the first monoclonal antibody and the second monoclonal antibody may be referred to as a primary antibody and a secondary antibody, respectively.

Regarding the reactivity between the monoclonal antibody and the antigen, "reacting", "recognizing", and "binding" are synonymously used.

Stating that the antibody of the present invention "does not react with" a certain compound means that the antibody does not substantially react with the certain compound and, for example, in the examples described later, this means less than 0.1 Abs in measurement using a plate reader of solid phase-antigen ELISA.

While the antigen for obtaining the first monoclonal antibody may be any glycated human hemoglobin, typically, HbA1c is preferably used, and HbA1c may be the whole of HbA1c or a portion thereof. In the examples described later, HbA1c was used as glycated human hemoglobin and HbA0 was used as non-glycated hemoglobin to acquire the first monoclonal antibody of the present invention. Therefore, the anti-glycated human hemoglobin monoclonal antibody in this case is particularly referred to as an anti-HbA1c monoclonal antibody.

The first monoclonal antibody recognized as an antigen for acquiring the second monoclonal antibody may be the whole monoclonal antibody or a portion thereof.

An antibody of the present invention can easily be produced by dissolving a substance in which a glycated peptide (f-VHLT, f-VHLTPEEKYYC: f means fructosylated) is bound to a carrier protein as an antigen (hereinafter referred to as a glycated peptide-binding protein) in a solvent such as phosphate buffered saline and administering this solution to an animal for immunization. The carrier protein may be bovine serum albumin, keyhole-limpet hemocyanin, egg albumin, etc. The immunization may be performed using an emulsion after adding an appropriate adjuvant to the solution as required. The adjuvant may be a widely used adjuvant, such as water-in-oil emulsion, water-in-oil-in-water emulsion, oil-in-water emulsion, liposome, or aluminum hydroxide gel as well as a protein or peptidic substance derived from biogenic components. For example, Freund's incomplete or complete adjuvant can preferably be used. Although not particularly limited, it is desired that the administration route, administered dose, and administration time of the adjuvant are appropriately selected such that a desired immune response can be enhanced in an animal to be immunized by the antigen.

Although not particularly limited, the type of the animal used for the immunization is preferably a mammal and can be a mouse, rat, and rabbit, and a mouse is more preferable. The animal may be immunized in accordance with a common technique, e.g., the immunization can be achieved by subcutaneously, intracutaneously, intravenously, or intraperitoneally injecting the animal with a solution of an antigen, preferably a mixture with the adjuvant. Since an immune response is generally different depending on a type and strain of the animal to be immunized, it is desirable that an immunization schedule is appropriately set depending on the animal to be used. The antigen administration is preferably repeated several times after initial immunization.

Although the following operations are continuously performed when a monoclonal antibody is obtained, the operation is not limited thereto, and a method of producing the monoclonal antibody itself can be performed according to the method described in, for example, Antibodies, A Laboratory Manual (Cold Spring Harbor Laboratory Press, (1988)).

After final immunization, hybridomas can be produced by extracting spleen or lymph node cells, which are antibody-producing cells, from an immunized animal and by fusing the cells with myeloma cells having high proliferative ability. Cells having high antibody-producing ability (quantitative and qualitative) are preferably used for the cell fusion, and the myeloma cells are preferably compatible with the animal from which the antibody-producing cells to be fused are derived. The cell fusion can be performed in accordance with a method known in the art, and a polyethylene glycol method, a method using Sendai virus, or a method utilizing electric current can be employed. The obtained hybridomas can be proliferated in accordance with a known method, and the desired hybridomas can be selected while confirming a property of a produced antibody. The hybridomas can be cloned by a well-known method such as a limiting dilution or soft agar method.

A method for acquiring the first monoclonal antibody will be described. The hybridoma producing the first monoclonal antibody can efficiently be selected in consideration of the conditions under which the produced antibody is actually used in measurement. For example, the first monoclonal antibody can be obtained by selecting a hybridoma producing an antibody reacting with HbA1c by an ELISA method, an RIA method, etc. Specifically, the first monoclonal antibody can be obtained by selecting a hybridoma producing a monoclonal antibody exhibiting a high reactivity to HbA1c and not reactive with HbA0 by a solid phase-antigen ELISA method in which monoclonal antibodies in a culture supernatant are first allowed to react with an immobilized glycated peptide-binding protein and then allowed to react with immobilized HbA0.

A monoclonal antibody having a desired property can be produced by mass cultivation of the hybridoma selected as described above. Although a method of mass cultivation is not particularly limited, examples thereof can include a method of producing the monoclonal antibody in culture media by cultivating the hybridoma in appropriate culture media and a method of producing the antibody in abdominal dropsy by injecting the hybridoma into the abdominal cavity of a mammal for proliferation. The monoclonal antibody can be purified by appropriately combining, for example, anion exchange chromatography, affinity chromatography, an ammonium sulphate fractionation method, a PEG fractionation method, and an ethanol fractionation method.

The selection of the hybridoma producing the second monoclonal antibody is performed by selecting a hybridoma producing an antibody reacting with the anti-glycated hemoglobin monoclonal antibody that is the first monoclonal antibody. To reduce non-specific reactions, preferably, a step of selecting an antibody having a reactivity to human IgG lower than that to mouse IgG is added after the step described above.

Specifically, the second monoclonal antibody can be obtained through two steps, i.e., a step of first selecting a hybridoma exhibiting a high reactivity to an anti-glycated hemoglobin monoclonal antibody from monoclonal antibodies in a culture supernatant by a solid phase-antigen ELISA method etc., and a step of selecting an antibody having a reactivity to human IgG lower than that to mouse IgG.

The antibody of the present invention can be a whole antibody molecule as well as a functional fragment of an antibody having an antigen-antibody reaction activity and can be an antibody obtained through an immunization step of an animal as described above or obtained by a gene recombination technique, or a chimeric antibody. Examples of the functional fragment include F(ab')₂ and Fab, and these functional fragments can be produced by treating the antibody obtained as described above with a proteolytic enzyme (e.g., pepsin or papain).

In addition, the second monoclonal antibody of the present invention can be used as an immobilized (solid phase) antibody immobilized on an insoluble carrier or as a labeled antibody labeled with a commonly-used labeling substance well known to those skilled in the art described later. For example, the immobilized antibody can be produced by causing an insoluble carrier to physically adsorb or chemically bind to a monoclonal antibody (a suitable spacer may exist therebetween). The insoluble carrier can be made of a polymer base material such as a polystyrene resin, an inorganic base material such as glass, and a polysaccharide base material such as cellulose and agarose, and the shape thereof is not particularly limited and can be selected from any shapes such as a plate shape (e.g., microplate and membrane), a bead or particle shape (e.g., latex particles and colloidal magnetic particles), and a tubular shape (e.g., test tube).

By labeling the second monoclonal antibody of the present invention with a labeling substance that can bind to the antibody to form a labeled antibody, glycated human hemoglobin (%) in a sample can be measured. A labeled antibody, labeled protein A, labeled protein G, etc. that can bind to the second monoclonal antibody of the present invention are also usable. Examples of the labeling substance include enzymes, fluorescent substances, chemiluminescent substances, biotin, avidin, radioisotopes, colloidal gold particles, or colored latex. A method for binding the labeling substance and the antibody can be a method such as a glutaraldehyde method, a maleimide method, a pyridyl disulfide method, or a periodic acid method, which can be used by those skilled in the art, and the types of the immobilized and labeled antibodies and the methods for producing the antibodies are not limited to these examples. For example, when an enzyme such as peroxidase or alkali phosphatase is used as the labeling substance, the enzyme activity can be measured by using a specific substrate of the enzyme (e.g., O-phenylenediamine or 3,3',5,5'-tetramethylbenzidine when the enzyme is horseradish peroxidase, p-nitrophenyl phosphate in the case of ALP), and when biotin is used as the labeling substance, at least avidin or enzyme-modified avidin is typically reacted therewith.

is In this description, an "insoluble carrier" is represented as a "solid phase", and physically or chemically supporting an antigen or antibody with an insoluble carrier or the supporting state may be represented as "immobilizing", "immobilized", "solid phased", "sensitization", "adsorption" and "binding". The term "detection" or "measurement" as used herein has meaning including the existence proof and/or the quantitation of glycated human hemoglobin.

A "sample" of an object to be detected in the assay method using the antibody of the present invention may be any human sample that may contain glycated human hemoglobin, and examples thereof can mainly include body fluids derived from living bodies (organisms). Specifically, the sample is a blood cell or whole blood. These samples may directly be used or may also be used after being diluted with a specimen diluent or subjected to other pretreatments.

### <Particle Agglutination Immunoassay Method: (LTIA method)>

For the assay reagent for detecting glycated human hemoglobin (%) present in the sample, at least the following elements are required:
(a) an anti-glycated human hemoglobin monoclonal antibody;
(b) a monoclonal antibody reacting with the anti-glycated human hemoglobin monoclonal antibody and not reacting with human IgG; and
(c) insoluble carrier particles wherein the insoluble carrier is latex

The latex particles used can appropriately be selected in terms of diameter and type so as to obtain desired performance such as high sensitivity. The latex particles may be any particles suitable for adsorption and binding of the antigen HbA1c. Examples thereof include polystyrene, styrene-sulphonic acid (sulfonate) copolymer, styrene-methacrylic acid copolymer, acrylicnitrile-butadiene-styrene copolymer, vinyl chloride-acrylic acid ester copolymer, and vinyl acetate-acrylic acid ester copolymer. Although the shape of the a monoclonal antibody reacting with the anti-glycated human hemoglobin monoclonal antibody and not reacting with human IgG latex particles is not particularly limited, the average particle diameter preferably has a size sufficient for detection with the naked eye, or optical detection, of aggregates generated as a result of an immune reaction between HbA1c on the surface of the latex particles and the antibody and an agglutination reaction between the antibodies. The average particle diameter is preferably 0.02 to 1.6 µm, particularly preferably 0.03 to 0.5 µm. Particles made of a material such as metal colloid, gelatin, liposome, microcapsules, silica, alumina, carbon black, metal compound, metal, ceramics, or magnetic material can be used instead of the latex particles. These particles may contain a fluorescent substance. The insoluble carrier of the present invention is preferably unsensitized. The reagent containing the latex particles of the present invention appropriately contains a buffer component (buffer solution).

The buffer solution usable in the present invention may be any generally used buffer solution, and examples thereof include tris-hydrochloric acid, boric acid, phosphoric acid, acetic acid, citric acid, succinic acid, phthalic acid, glutaric acid, maleic acid, glycin, and salts thereof, and Good's buffers such as MES, Bis-Tris, ADA, PIPES, ACES, MOPSO, BES, MOPS, TES, and HEPES.

The reagents of the present invention are two reagents (two-step method). In the case of two reagents, the reagents are preferably divided into a first reagent containing an insoluble carrier, wherein the insoluble carrier is latex, and a second reagent containing two types of monoclonal antibodies.
1) an anti-glycated human hemoglobin monoclonal antibody; and
2) a monoclonal antibody reacting with the anti-glycated human hemoglobin monoclonal antibody and not reacting with human IgG.

In the measurement of latex agglutination, glycated human hemoglobin (%) can be measured by optically or electrochemically observing a degree of generated agglutination. Examples of a method for optical observation include methods for measuring scattered
*: 1) an anti-glycated human hemoglobin monoclonal antibody; and
2) a monoclonal antibody reacting with the anti-glycated human hemoglobin monoclonal antibody and not reacting with human IgG.
light intensity, absorbance, or transmitted light intensity with an optical instrument (endpoint method, rate method, etc.).

A measurement value of the absorbance, etc. obtained by measuring the sample is compared with a measurement value of the absorbance, etc. obtained by measuring a standard substance (sample with known glycated human hemoglobin (%)) to calculate a concentration (quantitative value) of an analyte contained in the sample. The measurement of the absorbance etc. of transmitted light or scattered light may be one-wavelength measurement or two-wavelength measurement (difference or ratio between two wavelengths). The measurement wavelength is typically selected from 400 nm to 800 nm.

The analyte in the sample of the present invention may be measured by a manual method or may be measured by using an apparatus such as a measuring apparatus. The measuring apparatus may be a general-purpose automated analyzer or a dedicated measuring apparatus (dedicated machine). The dedicated measuring apparatus may be the following inspection device using a microchannel for mixing and reacting a sample with a reagent to perform detection. Specifically, a reaction cassette rotationally operated around a horizontal rotation axis is employed, and this reaction cassette is equipped with a microchannel and an injection hole communicating with the microchannel and introducing a liquid sample into the microchannel and includes a means for easily introducing a diluent. The reaction channel is equipped with a reagent area in which an analytical reagent is incorporated, and a means for disturbing a flow of the liquid sample due to gravity along the microchannel such that the liquid sample is brought into contact with the analytical reagent and that the liquid sample is stirred together with the analytical reagent to sufficiently promote a predetermined reaction. The device is arranged such that the reaction cassette configured in this way is rotated and vibrated to allow the liquid sample to flow through the microchannel and come into contact with the analytical reagent and to cause the liquid sample to be stirred together with the analytical reagent so as to promote a predetermined reaction and measure a detectable reaction in the liquid sample.

The measurement of the present invention is conducted by a method performed through a two-step method (two-reagent method).

The present invention enables accurate immunoassay of glycated human hemoglobin (%) even when the human specimen contains not only normal human (hemoglobin but also abnormal human emoglobin such as HbS and HbC, which are mutants. Therefore, the assay method of the present invention is also a method for reducing a deviation of a glycated human hemoglobin (%) measurement value in a sample containing abnormal human hemoglobin and is also a method for suppressing a false high value in a glycated human hemoglobin (%) measurement value in a sample containing abnormal human hemoglobin.

### EXAMPLES

### [Example] Production of Assay Reagent of the Present Invention

### 1. Acquisition of the First Monoclonal Antibody

An anti-HbA1c monoclonal antibody was produced as the first monoclonal antibody of the present invention as follows.

### (1) Preparation of Purified HbA0 and Purified HbA1c

HbA0 and HbA1c were purified from human erythrocyte hemolysate by ion exchange chromatography using Bio-Rex70 (Bio-Rad) described in a non-patent document (Melisenda J. McDonald et. al., JBC, 253(7), 2327-2332, 1978) and was used in subsequent experiments.

### (2) Preparation of Various Peptides and Glycated Peptides

Peptides having two types of amino sequences (VHLTC (SEQ ID NO: 1) and VHLTPEEKYYC (SEQ ID NO: 2): alphabet indicates one-letter notation for amino acids) were synthesized and purified by the Fmoc method using an automated peptide synthesizer. It was confirmed by HPLC that the purity of each of the peptides was 95% or more. It was confirmed by mass spectrometry (MALDI-TOF method) that the molecular weight of each of the peptides was the same as the theoretical value.

The two types of peptides were glycated by the method described in Japanese Laid-Open Patent Publication No. 61-172064 to purify glycated peptides (f-VHLTC and f-VHLTPEEKYYC: f means fructosylation). Specifically, each of the peptides having the sequences was reacted with glucose in anhydrous pyridine to synthesize a glycated peptide, which was purified by HPLC. It was confirmed by mass spectrometry (MALDI-TOF method) that the molecular weight of each of the glycated peptides was the same as the theoretical value, i.e., the molecular weight obtained by adding 162 to the molecular weight of each of the peptides.

### (3) Acquisition of the First Monoclonal Antibody

The anti-HbA1c monoclonal antibody serving as the first monoclonal antibody was produced as follows. First, the glycated peptide (f-VHLTPEEKYYC) synthesized in (2) was bound to keyhole-limpet hemocyanin, and this was used as an immunogen to immunize a mouse. Hybridomas were screened by selecting 6 strains reacting with a glycated peptide-binding protein and not reacting with the purified HbA0 in solid phase-antigen ELISA. After cloning, monoclonal antibodies (antibodies 68207, 68208, 68210, 68211, 68213, 68214) reacting with purified HbA1c and not reacting with purified HbA0 in solid phase-antigen ELISA were finally obtained.

### 2. Acquisition of the Second Monoclonal Antibody

A monoclonal antibody to the anti-HbA1c monoclonal antibody was produced as the second monoclonal antibody of the present invention as follows.

### (1) Immunization

The three different mouse anti-HbA1c monoclonal antibodies (68210, 68211, 68213: prepaired by Sekisui Medical) obtained in 1. were used as immunogens, and rats were used as hosts for performing immunization by a conventional method. Final immunization was performed 3 to 4 days before cell fusion, and spleen cells and lymph node cells were collected and fused with myeloma cells (SP2/O) by electrofusion or PEG. The fused cells were cultured on a 96-well plate, and the culture supernatant was collected after 7 to 8 days from the cell fusion and screened as described below.

### (2) Screening

In the screening of hybridomas, a strain reacting with mouse IgG and not reacting with human IgG was selected by solid phase-antigen ELISA.

### (2-1) Solid Phase-Antigen ELISA

(i) To a 96-well plate for ELISA, 50 µL/well of mouse anti-HbA1c monoclonal antibody (68208 or 68214) or human IgG diluted with PBS to 1 µg/mL was dispensed as a solid-phased antigen at room temperature and allowed to stand for 2 hours.
(ii) After washing 3 times with 0.05 % Tween20-PBS (PBST) (400 µL/well), 1 % BSA-PBST (100 µL/well) was dispensed as a blocking solution and allowed to stand at room temperature for 1 hour.
(iii) After removing the blocking solution, 50 µL/well of the culture supernatant diluted 2-fold was dispensed and allowed to stand at room temperature for 1 hour.
(iv) After washing 3 times, 50 µL/well of 10000-fold diluted HRP-labeled goat anti-rat polyclonal antibody was dispensed and allowed to stand at room temperature for 1 hour.
(v) After washing three times, 50 µL/well of an OPD coloring solution was dispensed and allowed to stand at room temperature for 10 minutes.
(vi) After 50 µL/well of a stop solution was dispensed to stop the reaction, the absorbance was measured with a plate reader (Abs. 492 nm).

### (2-2) Results

A total of 13 strains (S07201R to S072013R) of hybridomas were acquired (Table 1).

**[Table 1]**

| | | | (Abs.) |
|---|---|---|---|
| immunogen | clone name | antigen for screening | |
| | | mouseIgG | humanIgG |
| 68210 | S07201R | 0.596 | 0.074 |
| | S07202R | 0.521 | 0.077 |
| 68211 | S07203R | 2.543 | 0.063 |
| | S07204R | 2.274 | 0.062 |
| | S07205R | 2.819 | 0.066 |
| | S07206R | 2.475 | 0.083 |
| | S07207R | 2.729 | 0.066 |
| 68213 | S07208R | 0.486 | 0.059 |
| | S07209R | 0.544 | 0.062 |
| | S07210R | 0.926 | 0.095 |
| | S07211R | 0.529 | 0.077 |
| | S07212R | 0.879 | 0.076 |
| | S07213R | 0.992 | 0.072 |

### (3) Confirmation of Reactivity of Acquired Antibody

The hybridomas selected by screening were made monoclonal by the limiting dilution method and administered to the abdominal cavity of mice by a conventional method to obtain ascites. Antibodies were purified from the obtained ascites, and the reactivity was confirmed by solid phase-antigen ELISA.

### (3-1) Solid Phase-Antigen ELISA

The same method as (2) Screening was used except that 68210 and 68213 were used as solid-phased antigens.

### (3-2) Results

The obtained 13 types of antibodies exhibited a reactivity to mouse IgG (anti-mouse IgG monoclonal antibody) stronger than that to human IgG for any of the solid-phased antigens.

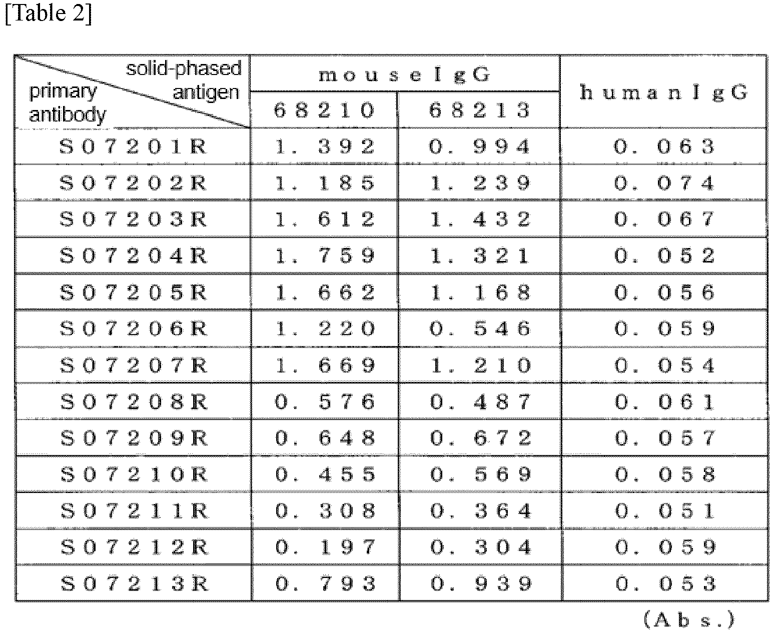

### 3. Immunological Assay Method

### (1) Example Formulation

(i) The composition of the first reagent is shown below.
   10 mM EPPS (pH 8.0)
   Latex particles (average particle diameter: about 120 nm)
(ii) The composition of the second reagent is shown below.
   10 mM Bis-Tris (pH 6.0)
   Primary antibody (0.1 mg/mL mouse anti-HbA1c monoclonal antibody) (Table 3)
   Secondary antibody (0.1 mg/mL rat anti-mouse IgG monoclonal antibody) (Table 3)
   500 mM NaCl
   0.05% Tween-20
   0.09% sodium azide

**[Table 3]**

| number | primary antibody | secondary antibody |
|---|---|---|
| Example 1 | 68210 | S07201R |
| Example 2 | 68211 | S07201R |
| Example 3 | 68213 | S07201 R |
| Example 4 | 68207 | S07205R |
| Example 5 | 68211 | S07204R |
| Example 6 | 68211 | S07206R |
| Example 7 | 68211 | S07210R |
| Example 8 | 68211 | S07211R |
| Example 9 | 68211 | S07212R |
| Example 10 | 68213 | S07203R |
| Example 11 | 68213 | S07210R |
| Example 12 | 68213 | S07211R |
| Example 13 | 68213 | S07212R |
| Example 14 | 68213 | S07213R |
| Example 15 | 68214 | S07209R |
| Example 16 | 68214 | S07210R |
| Example 17 | 68211 | S07203R |

### (2) Assay Method

A Hitachi 7170 automated analyzer was used for measurement. After 6.3 µL of a specimen and 150 µL of the first reagent were added to a reaction cell and reacted at 37 °C for 5 minutes, 50 µL of the second reagent is further added to the reaction cell and reacted at 37 °C for 5 minutes, and an amount of change in absorbance was measured by a 2-point end method (photometric points 19-34) at a main wavelength of 660 nm and a sub-wavelength of 800 nm. A calibration curve was prepared by using Determiner calibrator HbA1c for liquid reagent manufactured by Kyowa Medex Co., Ltd. as a reference standard by a method specified by the manufacturer.

### (3) Comparative Example

(i) Comparative Example 1: HPLC method (Adams HA-8180T manufactured by ARKRAY)
   Glycated hemoglobin (%) of various specimens was measured by using the method specified by the manufacturer.
(ii) Comparative Example 2: LTIA Method Using Polyclonal Antibody as Secondary Antibody (1) (Determiner L HbA1c Manufactured by Kyowa Medex Co., Ltd.)
   Glycated hemoglobin (%) of various specimens was measured by using Determiner L HbA1c manufactured by Kyowa Medex Co., Ltd. by the method specified by the manufacturer. For the measurement, the Hitachi 7170 automated analyzer was used.

### (4) Reference Example: LTIA Method Using Polyclonal Antibody as Secondary Antibody (2) (prepaired by Sekisui Medical Co., Ltd.)

Glycated hemoglobin (%) of various specimens was measured by using the first reagent described in Example 2, and the second reagent having the same composition as Example 2 except that 0.06 mg/mL of 68210 serving as the primary antibody, and 0.05 mg/mL of the goat anti-mouse IgG polyclonal antibody (Fc-specific antibody: manufactured by Tanpaku Seisei Kougyou) serving as the secondary antibody were added. For the measurement, the Hitachi 7170 automated analyzer was used.

### [Test Example 1] Measurement of Glycated Hemoglobin (%) of Blood Cell Specimen

### (1) Measurement of Glycated Hemoglobin (%) of Normal Hb Specimens

It was confirmed that glycated hemoglobin (%) of normal specimens was measurable with high accuracy in Reference Example and Examples 1 to 17 as compared to Comparative Example 2 using the ready-made product (Determiner L HbA1c manufactured by Kyowa Medex Co., Ltd.).

Any normal blood specimen (obtained from a volunteer with consent) collected with an EDTA blood collection tube was centrifuged at 800 G for 5 minutes, and the glycated hemoglobin (%) of the obtained lower blood cell layer diluted 100-fold with purified water was measured. Table 4 shows a correlation equation and a correlation coefficient of the reagents compared with Determiner L HbA1c when 50 specimens were measured.

The correlation coefficient was favorable in all cases, and it was demonstrated that glycated hemoglobin (%) can be measured with high accuracy in normal specimens in Reference Examples and Examples 1 to 17.

**[Table 4]**

| **number** | **correlation coefficient r²** |
|---|---|
| Reference Example | 0.9963 |
| Example 1 | 0.9980 |
| Example 2 | 0.9949 |
| Example 3 | 0.9904 |
| Example 4 | 0.9904 |
| Example 5 | 0.9975 |
| Example 6 | 0.9930 |
| Example 7 | 0.9967 |
| Example 8 | 0.9959 |
| Example 9 | 0.9971 |
| Example 10 | 0.9958 |
| Example 11 | 0.9970 |
| Example 12 | 0.9972 |
| Example 13 | 0.9974 |
| Example 14 | 0.9975 |
| Example 15 | 0.9890 |
| Example 16 | 0.9959 |
| Example 17 | 0.9935 |

### (2) Measurement of Glycated Hemoglobin (%) of Abnormal Hb Specimens

Glycated hemoglobin (%) of commercially available abnormal Hb specimens (purchased from Bizcom-Japan) was measured. These are blood cell specimens cryopreserved at -70 °C or lower after separating the blood cell layer by centrifugation after blood collection.

### (2-1) Measurement of Glycated Hemoglobin (%) of HbS-Containing Specimen

Adams HA-8180T manufactured by ARKRAY, i.e., the HPLC method described in Comparative Example 1, can detect abnormal Hb, HbS and HbC, in blood cells and whole blood specimens in Variant mode. HbS (%) of the blood cell specimen containing HbS used was 33.2 %. The glycated hemoglobin (%) of the specimen containing HbS described above was measured by the HPLC method and was 6.2 %. Table 5 shows differences (glycated hemoglobin (%)) of the glycated hemoglobin (%) of the specimen containing HbS described above measured with the reagents of Examples 1 to 10, Comparative Example 2, and Reference Example from the glycated hemoglobin (%) measured by the HPLC method.

In Comparative Example 2 using the polyclonal antibody as the secondary antibody, the measurement value was 6.9 %, which was 0.7 % higher than the HPLC method. Even in Reference Example in which the polyclonal antibody was combined as the secondary antibody, the measurement value was 6.7 %, which was 0.5 % higher than the HPLC method.

On the other hand, in Examples 1 to 10 in which the monoclonal antibody of the present disclosure was combined as the secondary antibody, the measurement values were 5.9 % to 6.4 %, and the difference from the HPLC method was -0.2 % to 0.2 %. It can be said that the measurement accuracy was improved when the monoclonal antibody was used as the secondary antibody in the blood cell specimen containing the mutant Hb, HbS.

**[Table 5]**

| assay method | | | difference from Comparative Example 1 (HPLC method) in glycated hemoglobin (%) | |
|---|---|---|---|---|
| Comparative Example 1 | | HPLC method | 0.0 | |
| Comparative Example 2 | | Determiner L HbA1c | 0.7 | |
| Reference Example | own reagent: polyclonal antibody | | 0.5 | |
| Example 1 | own reagent: monoclonal antibody | | -0.1 | |
| Example 2 | own reagent: monoclonal antibody | | -0.2 | |
| Example 3 | own reagent: monoclonal antibody | | -0.1 | |
| Example 4 | own reagent: monoclonal antibody | | -0.1 | |
| Example 5 | own reagent: monoclonal antibody | | 0.2 | |
| Example 6 | own reagent: monoclonal antibody | | 0.1 | |
| Example 7 | own reagent: monoclonal antibody | | 0.1 | |
| Example 8 | own reagent: monoclonal antibody | | 0.0 | |
| Example 9 | own reagent: monoclonal antibody | | 0.0 | |
| Example 10 | own reagent: monoclonal antibody | | 0.2 | |

### (2-2) Measurement of Glycated Hemoglobin (%) of HbC-Containing Specimen

HbC (%) of the blood cell specimen containing HbC used was measured by the HPLC method and was 31.8 % to 40.9 %.

The glycated hemoglobin (%) of the specimen containing HbS described above was measured by the HPLC method and was 4.8 to 5.9 %.

Table 6 shows differences (glycated hemoglobin (%)) of the glycated hemoglobin (%) of the specimen containing HbC described above measured with the reagents of Comparative Example 2, Reference Example, and Example 1 from the glycated hemoglobin (%) measured by the HPLC method.

When the specimen was measured in Comparative Example 2 using the polyclonal antibody as the secondary antibody, the difference from the HPLC method was as high as 0.9 to 3.1 %, so that the glycated hemoglobin (%) of the blood cell specimen containing HbC was not accurately measurable with the reagent of Comparative Example 2. In Reference Example, the difference from the HPLC method was 0.5 to 1.7 %, so that the glycated hemoglobin (%) was measurable more accurately than in Comparative Example 2.

In Example 1 in which the monoclonal antibody of the present disclosure was combined as the secondary antibody, the difference from the HPLC method was -0.4 to - 0.2 %, so that the glycated hemoglobin (%) was measurable with high accuracy even in the blood cell specimen containing HbC.

**[Table 6]**

| specimen number | HbC % | difference from Comparative Example 1 (HPLC method) in glycated hemoglobin (%) | | |
|---|---|---|---|---|
| | | Comparative Example 2 | Reference Example | Example 1 |
| | | Determiner IL HbA1c | own reagent: polyclonal antibody | own reagent: monoclonal antibody |
| HbC-1 | 31.8 | 0.9 | 0.5 | -0.4 |
| HbC-2 | 35.7 | 1.7 | 0.7 | -0.2 |
| HbC-3 | 40.9 | 2.9 | 1.7 | -0.2 |
| HbC-4 | 35.2 | 1.9 | 1.0 | -0.3 |
| HbC-5 | 40.2 | 2.3 | 0.8 | -0.4 |
| HbC-6 | 35.2 | 2.0 | 1.1 | -0.2 |
| HbC-7 | 40.5 | 3.1 | 1.3 | -0.2 |
| HbC-8 | 35.6 | 2.2 | 1.0 | -0.3 |

Table 7 shows the results of measurement of the specimen number HbC-1 with the reagents of Examples 8 to 17. The measurement results of Comparative Examples 1 and 2, Reference Example, and Example 1 are the same as those in Table 6. The glycated hemoglobin (%) of HbC-1 was 5.8 % when measured by the HPLC method of Comparative Example 1. When this specimen was measured in Comparative Example 2 in which the polyclonal antibody was used as the secondary antibody, the difference in the measurement value from the HPLC method was 0.9 %, which was higher as compared to Reference Example and the method of the present invention, so that the glycated hemoglobin (%) of the specimen containing HbC was not accurately measurable with the reagent of Example 2. In Reference Example, the difference in the measurement value from the HPLC method was 0.5 %, so that the glycated hemoglobin (%) was measurable more accurately than in Comparative Example 2. Further, in Examples 8 to 17 in which the monoclonal antibody of the present invention was combined as the secondary antibody, the difference in the measurement values from the HPLC method was -0.1 to 0.3 %, so that the glycated hemoglobin (%) was measurable with high accuracy even in the specimen containing HbC.

**[Table 7]**

| assay method | | | difference from Comparative Example 1 (HPLC method) in glycated hemoglobin (%) |
|---|---|---|---|
| Comparative Example 1 | | HPLC method | 0 |
| Comparative Example 2 | | Determiner L HbA1c | 0.9 |
| Reference Example | own reagent: polyclonal antibody | | 0.5 |
| Example 8 | own reagent: monoclonal antibody | | -0.1 |
| Example 9 | own reagent: monoclonal antibody | | 0.1 |
| Example 10 | own reagent: monoclonal antibody | | 0.2 |
| Example 11 | own reagent: monoclonal antibody | | 0.2 |
| Example 12 | own reagent: monoclonal antibody | | 0.0 |
| Example 13 | own reagent: monoclonal antibody | | -0.1 |
| Example 14 | own reagent: monoclonal antibody | | 0.3 |
| Example 15 | own reagent: monoclonal antibody | | 0.0 |
| Example 16 | own reagent: monoclonal antibody | | 0.2 |
| Example 17 | own reagent: monoclonal antibody | | 0.1 |

### [Test Example 2] Measurement of Glycated Hemoglobin (%) of Whole Blood Specimen

### (1) Measurement of Glycated Hemoglobin (%) of Normal Hb Specimens

It was confirmed that glycated hemoglobin (%) of normal whole blood specimens was measurable with high accuracy in Comparative Example 3 and Examples 3, 4, 8, 9, 11 to 13, and 15 as compared to the measurement value of glycated hemoglobin of the blood cell specimen of Comparative Example 2 using the ready-made product (Determiner L HbA1c manufactured by Kyowa Medex Co., Ltd.).

The glycated hemoglobin (%) was measured in any normal blood specimen collected with the EDTA blood collection tube used in the measurement of the normal blood specimen of Test Example 1 (1). Each specimen was used in the whole blood state without centrifugation and prepared by diluting the whole blood 50-fold with purified water. Table 8 shows a correlation coefficient for the measurement results of the whole blood specimens of the reagents when 50 specimens were measured, compared with the measurement value when the blood cell specimen was measured with the Determiner L HbA1c. In the reagent of Comparative Example 2, the correlation coefficient was the lowest as compared to when the other reagents were used, and glycated hemoglobin (%) was not accurately measurable for the whole blood specimens. On the other hand, in Reference Examples, and Examples 3, 4, 8, 9, 11 to 13, and 15, the correlation coefficient was favorable, and it was demonstrated that glycated hemoglobin (%) was measurable with high accuracy in the normal specimens of whole blood.

**[Table 8]**

| number | primary antibody | secondary antibody | correlation (correlation coefficient r²) with Comparative Example 2 (at the time of measurement of blood cell specimen) |
|---|---|---|---|
| Comparative Example 2 | Determiner L HbA1c | | 0.9683 |
| Reference Example | 68210 | polyclonal antibody | 0.9782 |
| Example 3 | 68213 | S07201R | 0.9831 |
| Example 4 | 68207 | S07205R | 0.9827 |
| Example 8 | 68211 | S07211R | 0.9865 |
| Example 9 | 68211 | S07212R | 0.9892 |
| Example 11 | 68213 | S07210R | 0.9856 |
| Example 12 | 68213 | S07211R | 0.9875 |
| Example 13 | 68213 | S07212R | 0.9876 |
| Example 15 | 68214 | S07209R | 0.9878 |

### (2) Measurement of Glycated Hemoglobin (%) of Abnormal Hb Specimens

Commercially available abnormal Hb specimens (purchased from Bizcom-Japan) were used for measurement. The specimens were whole blood specimens cryopreserved at -70 °C or lower after blood collection.

### (2-1) Measurement of Glycated Hemoglobin (%) of HbS-Containing Specimen

HbS (%) and glycated hemoglobin (%) of the whole blood specimen containing HbS used were measured by the HPLC method and were 28.8 to 37.1 % and 4.6 to 6.5 %, respectively.

Table 9 shows differences in glycated hemoglobin (%) of the measurement values measured with the reagents of Comparative Example 2, Reference Example, and Examples 3, 4, 11, 13, and 15 from the measurement value for the HPLC method.

The content measured with the reagent of Comparative Example 2 was 0.5 to 1.4 % higher than the HPLC method, so that the measurement accuracy was poor.

The content measured with the reagent of Reference Example was better than Comparative Example 2 although the content was 0.3 to 0.6 % higher than the HPLC method.

On the other hand, the measurement values measured with the reagents of Examples 3, 4, 11, 13, and 15 had a small difference of -0.1 to 0.3 % from the HPLC method, so that the measurement accuracy was favorable. When the monoclonal antibody of the present invention was used as the secondary antibody, the measurement accuracy was further improved as compared to when the polyclonal antibody was used as the secondary antibody (Reference Example).

**[Table 9]**

| specimen number | HbS content (%) | difference from Comparative Example 1 (HPLC method) in glycated hemoglobin (%) | | | | | | |
|---|---|---|---|---|---|---|---|---|
| | | Comparative Example 2 | Reference Example | Example 3 | Example 4 | Example 11 | Example 13 | Example 15 |
| HhS-2 | 28.8 | 1.1 | 0.4 | -0.2 | -0.1 | 0.0 | 0.0 | 0.0 |
| HbS-3 | 37.1 | 1.4 | 0.3 | 0.0 | 0.0 | -0.1 | -0.1 | -0.2 |
| HbS-4 | 33.5 | 0.5 | 0.5 | -0.1 | 0.1 | 0.1 | 0.0 | 0.0 |
| HbS-5 | 34.8 | 0.9 | 0.6 | 0.2 | 0.3 | 0.3 | 0.2 | 0.2 |
| HbS-6 | 32.7 | 0.6 | 0.5 | 0.0 | 0.1 | 0.1 | 0.0 | -0.1 |

### (2-2) Measurement of Glycated Hemoglobin (%) of HbC-Containing Specimen

HbC (%) and glycated hemoglobin (%) of the whole blood specimen containing the HbC used were measured by the HPLC method and were 34.9 % to 39.4 % and 5.0 to 5.5 %, respectively.

Table 10 shows differences (glycated hemoglobin (%)) of the measurement values measured with the reagents of Comparative Example 2, Reference Example, and Examples 3, 8, 9, 12, and 13 from the HPLC method.

First, the measurement value measured in Comparative Example 2 was significantly increased by 3.4 to 4.7 % from the HPLC method.

Reference Example was better than Comparative Example 2 although the value was 0.9 to 1.1 % higher than the HPLC method.

On the other hand, the reagents of Examples 3, 8, 9, 12, and 13 had a small difference of 0.1 to 0.4 % from the HPLC method, so that the measurement accuracy was favorable. It can be said that the measurement accuracy was improved when the monoclonal antibody of the present invention was used as the secondary antibody.

**[Table 10]**

| specimen number | HbC content (%) | difference from Comparative Example 1 (HPLC method) in glycated hemoglobin (%) | | | | | | |
|---|---|---|---|---|---|---|---|---|
| | | Comparative Example 2 | Reference Example | Example 3 | Example 8 | Example 9 | Example 12 | Example 13 |
| HbC-9 | 34.9 | 3.4 | 1.1 | 0.3 | 0.1 | 0.4 | 0.3 | 0.2 |
| HbC-10 | 39.4 | 4.7 | 0.9 | 0.4 | 0.1 | 0.3 | 0.1 | 0.2 |

## Claims

1. A glycated human hemoglobin (%) assay method for measuring glycated human hemoglobin (%) in a human sample, comprising steps of:
adsorbing the glycated human hemoglobin in the human sample to an insoluble carrier by bringing the human sample into contact with the insoluble carrier; wherein the insoluble carrier is latex;
forming a complex of an anti-glycated human hemoglobin monoclonal antibody, the glycated human hemoglobin, and the insoluble carrier by bringing the glycated human hemoglobin adsorbed to the insoluble carrier into contact with the anti-glycated human hemoglobin monoclonal antibody;
agglutinating the insoluble carrier by bringing the complex into contact with a monoclonal antibody reacting with the anti-glycated human hemoglobin monoclonal antibody and not reacting with human IgG; and
optically detecting a degree of agglutination.

2. The glycated human
hemoglobin (%) assay method according to claim 1, wherein the human sample is whole blood or blood cells.

3. Glycated human hemoglobin (%) assay reagents, comprising a first reagent and a second reagent, wherein
the first reagent contains an insoluble carrier, wherein the insoluble carrier is latex, and wherein the second reagent contains at least the following two types of monoclonal antibodies:
1) an anti-glycated human hemoglobin monoclonal antibody; and
2) a monoclonal antibody reacting with the anti-glycated human hemoglobin monoclonal antibody and not reacting with human !gG.

4. A glycated hemoglobin (%) assay kit comprising: glycated human hemoglobin (%) assay reagents according to claim 3.

5. A method for reducing a deviation of a measurement value of glycated human hemoglobin (%) in a human sample containing abnormal hemoglobin, the method comprising a step of bringing at least following two types of monoclonal antibodies, an insoluble carrier, wherein the insoluble carrier is latex, and the human sample into contact with each other:
1) an anti-glycated human hemoglobin monoclonal antibody; and
2) a monoclonal antibody reacting with the anti-glycated human hemoglobin monoclonal antibody and not reacting with human !gG.

6. A method for suppressing a false high value in measurement value of glycated human hemoglobin (%) in a human sample containing abnormal hemoglobin, the method comprising a step of bringing at least following two types of monoclonal antibodies, an insoluble carrier, wherein the insoluble carrier is latex, and the human sample into contact with each other:
1) an anti-glycated human hemoglobin monoclonal antibody; and
2) a monoclonal antibody reacting with the anti-glycated human hemoglobin monoclonal antibody and not reacting with human IgG.

## Patentansprüche

1. Glykiertes menschliches Hämoglobin (%) Assay-verfahren zur Messung von glykiertem menschlichem Hämoglobin (%) in einer menschlichen Probe, umfassend die folgenden Schritte:
Adsorbieren des glykierten menschlichen Hämoglobins in der menschlichen Probe an einen unlöslichen Träger, indem die menschliche Probe mit dem unlöslichen Träger in Kontakt gebracht wird; wobei der unlösliche Träger Latex ist;
Bilden eines Komplexes aus einem monoklonalen Antikörper gegen glykiertes menschliches Hämoglobin, dem glykierten menschlichen Hämoglobin und dem unlöslichen Träger, indem das glykierte menschliche Hämoglobin, das an den unlöslichen Träger adsorbiert ist, mit dem monoklonalen Antikörper gegen glykiertes menschliches Hämoglobin in Kontakt gebracht wird;
Agglutinieren des unlöslichen Trägers durch In-Kontakt-Bringen des Komplexes mit einem monoklonalen Antikörper, der mit dem monoklonalen Antikörper gegen glykiertes menschliches Hämoglobin reagiert und nicht mit menschlichem IgG reagiert; und
optisches Erfassen des Grades der Agglutination.

2. Glykiertes menschliches Hämoglobin (%) Assay-verfahren gemäß Anspruch 1, wobei die menschliche Probe Vollblut oder Blutzellen ist.

3. Glykiertes menschliches Hämoglobin (%) Testreagenzien, umfassend ein erstes Reagenz und ein zweites Reagenz, wobei das erste Reagenz einen unlöslichen Träger enthält, wobei der unlösliche Träger Latex ist, und wobei das zweite Reagenz mindestens die folgenden zwei Arten von monoklonalen Arbeitskörpern enthält:
1) einen monoklonalen Antikörper gegen glykiertes menschliches Hämoglobin; und
2) einen monoklonalen Antikörper, der mit dem monoklonalen Antikörper gegen glykiertes menschliches Hämoglobin reagiert und nicht mit menschlichem IgG reagiert.

4. Glykiertes menschliches Hämoglobin (%) Assay-kit umfassend: glykiertes menschliches Hämoglobin (%) Testreagenzien gemäß Anspruch 3.

5. Verfahren zum Verringern einer Abweichung eines Messwerts von glykiertem menschlichem Hämoglobin (%) in einer menschlichen Probe, die abnormales Hämoglobin enthält, wobei das Verfahren einen Schritt umfasst, bei dem mindestens die folgenden zwei Arten von monoklonalen Antikörpern, ein unlöslicher Träger, wobei der unlösliche Träger Latex ist, und die menschliche Probe in Kontakt gebracht werden:
1) einen monoklonalen Antikörper gegen glykiertes menschliches Hämoglobin; und
2) einen monoklonalen Antikörper, der mit dem monoklonalen Antikörper gegen glykiertes menschliches Hämoglobin reagiert und nicht mit menschlichem IgG reagiert.

6. Verfahren zur Unterdrückung eines falsch hohen Wertes im Messwert des glykierten menschlichen Hämoglobins (%) in einer menschlichen Probe, die abnormales Hämoglobin enthält, wobei das Verfahren einen Schritt umfasst, bei dem mindestens die folgenden zwei Arten von monoklonalen Antikörpern, einem unlöslichen Träger, wobei der unlösliche Träger Latex ist, und der menschlichen Probe in Kontakt gebracht werden:
1) einen monoklonalen Antikörper gegen glykiertes menschliches Hämoglobin; und
2) einen monoklonalen Antikörper, der mit dem monoklonalen Antikörper gegen glykiertes menschliches Hämoglobin reagiert und nicht mit menschlichem IgG reagiert.

## Revendications

1. Procédé de dosage d'hémoglobine glyquée humaine (%) pour mesurer l'hémoglobine glyquée humaine (%) dans un échantillon humain, comprenant les étapes consistant à :
adsorber l'hémoglobine glyquée humaine dans l'échantillon humain sur un support insoluble en amenant l'échantillon humain en contact avec le support insoluble ; dans lequel le support insoluble est du latex ;
former un complexe d'un anticorps monoclonal anti-hémoglobine glyquée humaine, de l'hémoglobine glyquée humaine, et du support insoluble en amenant l'hémoglobine glyquée humaine adsorbée sur le support insoluble en contact avec l'anticorps monoclonal anti-hémoglobine glyquée humaine ;
agglutiner le support insoluble en amenant le complexe en contact avec un anticorps monoclonal réagissant avec l'anticorps monoclonal anti-hémoglobine glyquée humaine et ne réagissant pas avec l'IgG humaine ; et
détecter optiquement un degré d'agglutination.

2. Procédé de dosage d'hémoglobine glyquée humaine (%) selon la revendication 1, dans lequel l'échantillon humain est du sang total ou des cellules sanguines.

3. Réactifs de dosage d'hémoglobine glyquée humaine (%), comprenant un premier réactif et un second réactif, dans lesquels
le premier réactif contient un support insoluble, dans lesquels le support insoluble est du latex, et dans lesquels le second réactif contient au moins les deux types suivants d'anticorps monoclonaux :
1) un anticorps monoclonal anti-hémoglobine glyquée humaine ; et
2) un anticorps monoclonal réagissant avec l'anticorps monoclonal anti-hémoglobine glyquée humaine et ne réagissant pas avec l'IgG humaine.

4. Kit de dosage d'hémoglobine glyquée (%) comprenant : des réactifs de dosage d'hémoglobine glyquée humaine (%) selon la revendication 3.

5. Procédé de réduction d'un écart d'une valeur de mesure d'hémoglobine glyquée humaine (%) dans un échantillon humain contenant de l'hémoglobine anormale, le procédé comprenant une étape consistant à amener au moins deux types suivants d'anticorps monoclonaux, un support insoluble, dans lequel le support insoluble est du latex, et l'échantillon humain en contact les uns avec les autres :
1) un anticorps monoclonal anti-hémoglobine glyquée humaine ; et
2) un anticorps monoclonal réagissant avec l'anticorps monoclonal anti-hémoglobine glyquée humaine et ne réagissant pas avec l'IgG humaine.

6. Procédé de suppression d'une fausse valeur élevée dans une valeur de mesure d'hémoglobine glyquée humaine (%) dans un échantillon humain contenant de l'hémoglobine anormale, le procédé comprenant une étape consistant à amener au moins deux types suivants d'anticorps monoclonaux, un support insoluble, dans lequel le support insoluble est du latex, et l'échantillon humain en contact les uns avec les autres :
1) un anticorps monoclonal anti-hémoglobine glyquée humaine ; et
2) un anticorps monoclonal réagissant avec l'anticorps monoclonal anti-hémoglobine glyquée humaine et ne réagissant pas avec l'IgG humaine.
